# EUROPÄISCHE PATENTSCHRIFT

(11) **EP 0 787 743 B1**
(45) Veröffentlichungstag und Bekanntmachung des Hinweises auf die Patenterteilung: **13.10.2004**
(21) Anmeldenummer: 96118320.9
(22) Anmeldetag: 15.11.1996
(51) Int. Cl.: C07K 16/28, C12N 5/18, A61K 39/395, G01N 33/53

(54) **A3C6E2, ein monoklonaler Antikörper spezifisch für den humanen Stammzellfaktor (SCF)-Rezeptor**
A3C6E2, a monoclonal antibody to the human stem cell factor (SCF)-receptor
A3C6E2, un anticorps monoclonal contre le récepteur humain du facteur de stimulation des cellules souches

(30) Priorität: 10.01.1996 DE 19600589
(43) Veröffentlichungstag der Anmeldung: 06.08.1997
(73) Patentinhaber: Eberhard-Karls-Universität Tübingen Universitätsklinikum, 72076 Tübingen (DE)
(72) Erfinder: Bühring, Hans-Jörg, Dr., 72076 Tübingen (DE)
(74) Vertreter: Otten, Hajo, Dr.-Ing.

(56) Entgegenhaltungen:
- WO-A-92/17505
- WO-A-92/21766
- J.BIOL.CHEM., Bd. 268, Nr. 6, 1993, Seiten 4399-4406, XP002029610 BLECHMANN ET AL.: "Soluble c-kit proteins and antireceptor monoclonal antibodies confine the binding site of the stem cell factor"
- TOHOKU J.EXP. MED., Bd. 178, Februar 1996, Seiten 187-198, XP000671052 MORITA ET AL.: "ISOLATION AND CHARACTERIZATION OF TWO MONOCLONAL ANTIBODIES THAT RECOGNIZE DIFFERENT EPITOPES OF THE HUMAN C-KIT RECEPTOR"

## Beschreibung

Die Erfindung betrifft einen monoklonalen Antikörper, der spezifisch an einen humanen Stammzellfaktor (SCF) - Rezeptor bindet.

Der Stammzellfaktor (SCF) ist ein Wachstumsfaktor, der die Proliferation der pluripotenten hämatopoetischen Blutstammzellen stimuliert. Er interagiert mit einem Rezeptor, dem CD117-Protein (Synonyme: SCF-Rezeptor oder c-kit-Rezeptor [Bühring et al. in Schlossman et al., (eds), Leucocyte Typing V, Oxford University Press, Oxford 1995, Seiten 1882-1888]), der bzw. das in der Plasmamembran der Blutstammzellen lokalisiert ist und von dem Proto-Onkogen c-kit kodiert wird (Zsebo et al., Cell 63: 213-224, 1990).

SCF selbst wird von verschiedenen natürlichen Zellen produziert und sezerniert und ist darüberhinaus mittlerweile als gentechnisch erzeugtes Rekombinationsprodukt aus beispielsweise E. coli im Handel erhältlich.

Die Blutstammzellen sind die undifferenzierten, unbegrenzt teilungsfähigen Ausgangszellen, aus denen sich die verschiedenen hochspezialisierten Blutzelltypen entwickeln. Sie sind verantwortlich für die natürliche, ständige Neubildung dieser verschiedenen Blutzelltypen und spielen eine wichtige Rolle bei der Entstehung und Entwicklung von Blutzellerkrankungen, insbesondere von Anämien, Leukämien und Lymphomen. Sie sind dazu bevorzugtes Zielobjekt bei der Diagnose und therapeutischen Behandlung solcher Krankheiten.

Die gezielte Identifikation, Isolierung und Beeinflussung der Blutstammzellen ist folglich von elementarer Bedeutung für eine möglichst schnelle und zuverlässige Diagnose und eine effektive, zielgenaue Therapie von Blutzellerkrankungen.

Als Vermittler für eine solche, zellulär zielgerichtete Behandlung kommt vor allem ein Antikörper in Betracht, der spezifisch an Blutstammzellen bindet. Ganz besonders eignet sich deshalb ein Antikörper, der das SCF - Rezeptorprotein CD117 als Antigen erkennt, da CD117 auf Stammzellen exprimiert ist.

Ein solcher Antikörper kann sowohl mit einfachen Nachweisreagenzien wie Fluoreszenzfarbstoffen oder radioaktiven Stoffen als auch mit speziellen therapeutisch wirksamen Reagenzien gekoppelt sein.

Da polyklonale Antikörper nur begrenzt verfügbar und nicht identisch reproduzierbar sind, sollte der Antikörper monoklonal sein.

In der WO 92/17505 ist der bisher einzige blockierende monoklonale Antikörper beschrieben, der den Stammzellfaktor-Rezeptor (SCF-R) bindet. Dieser Antikörper mit dem Namen SR-1 bindet spezifisch an das CD117-Protein und blockiert dadurch die Anbindung von SCF an diesen Rezeptor. Der Antikörper SR-1 gehört dem Isotyp IgG 2A an.

Mit diesem Antikörper SR-1 wurde einerseits ein sehr wirksames Hilfsmittel zur Identifizierung und Beeinflussung von Blutstammzellen und damit zur Diagnose und Therapie von Blutzellerkrankungen bereitgestellt. Andererseits bedeutete die bisherige Einzigartigkeit dieses Hilfsmittels, daß eine echte Kontrolle der damit gewonnenen Ergebnisse durch Parallelversuche mit einem vergleichbaren blockierenden monoklonalen Antikörper derselben oder einer sehr ähnlichen Spezifität nicht möglich war.

Der vorliegenden Erfindung liegt daher die Aufgabe zugrunde, mindestens einen weiteren blockierenden monoklonalen Antikörper bereitzustellen, der spezifisch an das humane SCF-Rezeptorprotein CD117 bindet.

Diese Aufgabe wird durch die Bereitstellung eines monoklonalen Antikörpers gelöst, der von Hybridomzellen produziert und freigsetzt wird, die unter der Nummer DSM ACC 2247 bei der Deutschen Sammlung von Mikroorganismen und Zellkulturen GmbH, DSM, nach den Vorschriften des Budapester Vertrags am 19.12.1995 hinterlegt worden sind. Der Antikörper ist mit der Bezeichnung A3C6E2 benannt. Er weist die Isotype IgG 1 auf und besitzt die Fähigkeit, die Bindung von SCF-Molekülen an den SCF-Rezeptor zu blockieren.

Mit dem erfindungsgemäßen Antikörper wurde ein zweiter monoklonaler Antikörper bereitgestellt, der standardisiert reproduzierbar ist und somit potentiell unbegrenzt hergestellt werden kann, und der spezifisch an das SR-1 Epitop auf dem SCF-Rezeptorprotein CD117 von humanen Blutstammzellen bindet.

Der erfindungsgemäße Antikörper ermöglicht eine gezielte Erkennung und Beeinflussung von Zellen, die eine extrazelluläre Domäne des SCF-Rezeptorproteins CD117 aufweisen. Er stellt damit ein zu dem bekannten Antikörper SR-1 alternatives Mittel für den Arzt und Forscher dar, um einerseits solche Zellen nachzuweisen, und zwar sowohl in der Zellkultur als auch im Patientenorganismus, und um andererseits diese Zellen ggf. zu manipulieren, entweder durch den Antikörper selbst oder durch daran gekoppelte, spezifische Reagenzien.

Mit der Bereitstellung dieses erfindungsgemäßen Antikörpers gegen das SCF-Rezeptorprotein CD117 ist es erstmals möglich, die mit dem bekannten Antikörper erhaltenen Testergebnisse wirksam zu kontrollieren und die Sicherheit ihrer Aussage maßgeblich zu erhöhen. Dasselbe gilt selbstverständlich ebenso im umgekehrten Fall mit dem erfindungsgemäßen Antikörper als primären Testantikörper und dem Antikörper SR-1 als Kontrollantikörper.

Die Erfindung betrifft ferner Hybridomzellen, die unter der Nummer DSM ACC 2247 bei der Deutschen Sammlung von Mikroorganismen und Zellkulturen GmbH, DSM, gemäß dem Budapester Vertrag hinterlegt sind und den Antikörper mit der Bezeichnung A3C6E2 produzieren.

Ein Verfahren zur Herstellung von Hybridomzellen, die einen Antikörper gegen das humane SCF-Rezeptorprotein CD117 synthetisieren und freisetzen, umfaßt die im Stand der Technik grundsätzlich geläufigen Schritte, wie sie bspw. von Bühring et al. in Hybridoma 1991, Band 10, Nr. 1, S. 77-78 beschrieben wurden:
1. Immunisierung oder Sensibilisierung eines Tieres, vorzugsweise einer Maus vom Balb/c-Stamm, mit dem Antigen bzw. Immunogen;
2. Gewinnung der antikörperproduzierenden Zellen, vorzugsweise der Milzlymphozyten dieses Tieres;
3. Fusionierung dieser antikörperproduzierenden Zellen mit einer stabilen, immortalisierten Zellinie, vorzugsweise einer Myelomzellinie, zu Hybridomzellen; und
4. Vereinzelung und Vermehrung (Klonierung) solcher Hybridomzellen, die einen Antikörper sezernieren, der an das Antigen bindet.

Das von den Erfindern weiterentwickelte Verfahren zeichnet sich nun dadurch aus, daß das Tier mit Zellen der undifferenzierten, megakaryoblastären Zellinie MOLM-1 immunisiert wird.

Dabei erwies sich als Vorteil, daß diese Zellinie eine starke Expression von SCF-Rezeptorprotein CD117 zeigt, wie sich während der zu dem Antikörper A3C6E2 führenden Versuche herausstellte.

Wenn Hybridomzellen gesucht werden, die stammzellspezifische Antikörper produzieren, ist es bei der Vereinzelung der Hybridomzellen günstig, wenn solche Hybridomzellen ausgewählt werden, die Antikörper mit einer Spezifität für Knochenmarkzellen produzieren, wobei es ferner günstig ist, wenn nur solche Hybridomzellen auf die Spezifität der von ihnen produzierten Antikörper mit Knochenmarkzellen getestet werden, bei denen zuvor nachgewiesen wurde, daß diese Antikörper eine schwache oder bevorzugt eine negative Reaktion mit peripheren Blutzellen zeigen.

Bei diesem Screening wird die Tatsache ausgenutzt, daß im Knochenmark verstärkt undifferenzierte Zellen einschließlich von hämatopoetischen Stammzellen vorkommen, die das von dem Antikörper zu erkennende Antigen aufweisen. Durch den Vortest auf negative Reaktion mit peripheren Blutzellen werden auf schnelle und einfache Weise, ohne daß viele Zellen vergebens getestet werden müssen, diejenigen Antikörper gewonnen, die selektiv an solche Antigene bindet, die charakteristisch für Knochenmarkzellen sind und nicht bzw. nur schwach von peripheren Blutzellen exprimiert werden. Mit anderen Worten, die Spezifität bei der Auswahl von stammzellspezifischen Antikörpern wird mit diesem Screeningverfahren auf einfache Weise deutlich erhöht.

Beim weiteren Screening werden günstigerweise solche Hybridomzellen ausgewählt, die einen Antikörper produzieren, der spezifisch an Fibroblasten bindet, die mit dem humanen c-kit Gen transfiziert sind.

Ein alternatives oder ergänzendes Screeningverfahren besteht darin, solche Hybridomzellen auszuwählen, die einen Antikörper produzieren, der die Bindung von SCF-Molekülen an die Blutstammzellen, d.h. an das SCF-Rezeptorprotein CD117 verhindern.

Andere Screeningverfahren wie die Immunpräzipitation von radioaktiv markiertem gereinigtem SCF-Rezeptorprotein oder der Enzyme linked immunosorbet assay, kurz ELISA, sind selbstverständlich auch möglich.

Der in diesen Tests eingesetzte SCF kann von verschiedenen Säugerarten stammen, wobei allerdings ein menschlicher SCF, insbesondere ein rekombinanter SCF, beispielsweise aus E. coli, bevorzugt ist.

Die Erfindung betrifft auch die In vitro-Verwendung des monoklonalen Antikörpers mit der Bezeichnung A3C6E2, wie er von den unter der Nummer DSM ACC 2247 bei der Deutschen Sammlung von Mikroorganismen und Zellkulturen GmbH, DSM, gemäß dem Budapester Vertrag hinterlegten Hybridomzellen produziert und freigesetzt wird, in einem Verfahren zur Diagnose von Tumoren, insbesondere von Leukämien und von Lymphomen.

Hier wird ausgenutzt, daß die Tumorzellen sich von den gesunden Blutzellen durch die Anzahl der SCF-Rezeptormoleküle in der Zellmembran unterscheiden. Ein erfindungsgemäßer Antikörper, der mit einem Nachweismittel, beispielsweise einem radioaktiven Marker gekoppelt ist, bindet dieses Nachweismittel indirekt an diese Zellen und ermöglicht damit die direkte Identifikation dieser Zellen, beispielsweise mit röntgendiagnostischen/szintigraphischen Methoden. Damit ist eine sehr frühe Tumordiagnose unter Umständen sogar in-vivo möglich.

Da der erfindungsgemäße Antikörper an den Rezeptor für den SCF bindet und damit an ein Protein, das eine zentrale Rolle bei der Regulation der Zellproliferation spielt, bietet er einen direkten Angriffspunkt für eine Manipulation, insbesondere eine Hemmung der Zellvermehrung.

Der erfindungsgemäße monoklonale Antikörper A3C6E2, wie er von den unter der Nummer DSM ACC 2247 bei der Deutschen Sammlung von Mikroorganismen und Zellkulturen GmbH, DSM, hinterlegten Hybridomzellen produziert und freigesetzt wird, kann deshalb auch zur therapeutischen Behandlung von malignen Blutzellen verwendet werden. Zu diesem Zwecke kann der Antikörper mit einem therapeutisch wirksamen Mittel gekoppelt sein und dadurch eine direkte und gezielte Beeinflussung oder gar Eliminierung der Tumorzellen ermöglichen.

Um die therapeutische bzw. die diagnostische Applikation des erfindungsgemäßen Antikörpers zu erleichtern, sollte der Antikörper mit entsprechend geeigneten Hilfssubstanzen in einer pharmazeutischen Zubereitung vermischt sein. Die Erfindung betrifft deshalb auch ein pharmazeutisches Mittel zur diagnostischen Behandlung sowie ein pharmazeutisches Mittel zur therapeutischen Behandlung von malignen Blutzellen, die jeweils den Antikörper A3C6E2 enthalten, wie er von den unter der Nummer DSM ACC 2247 bei der Deutschen Sammlung von Mikroorganismen und Zellkulturen GmbH, DSM, hinterlegten Hybridomzellen produziert und freigesetzt wird.

In dem pharmazeutischen Mittel kann der Antikörper A3C6E2 mit einem speziellen, gegen die Leukämiezellen und/oder die Lymphomzellen gerichteten Diagnostikum bzw. Therapeutikum gekoppelt sein.

Die Erfindung betrifft auch die In vitro-Verwendung eines erfindungsgemäßen Antikörpers zum Nachweis und/oder zur Isolierung von hämatopoetischen Zellen und ein dementsprechendes Verfahren. Dieses Verfahren zum Nachweis und/oder zur Isolierung von hämatopoetischen Zellen umfaßt die Schritte:
1) Inkubation einer Zellsuspension, die hämatopoetische Zellen enthält, mit einem Antikörper, der an hämatopoetische Zellen bindet, und
2) Abtrennung der den Antikörper bindenden Zellen von den übrigen Zellen,
und zeichnet sich dadurch aus, daß der Antikörper der erfindungsgemäße monoklonale Antikörper A3C6E2 ist.

Die Abtrennung erfolgt vorzugsweise mittels fluoreszenzaktivierter Zellsortierung (FACS), mittels Säulenchromatographie oder durch direkte Immunadhärens.

Bei der fluoreszenzaktivierten Zellsortierung (FACS) werden Zellen, die den SCF-Rezeptor tragen, mit dem erfindungsgemäßen monoklonalen Antikörper A3C6E2 gemischt, der zuvor direkt oder indirekt mit einem Fluoreszenzfarbstoff wie beispielsweise Fluoresceinisothiocyanat (FITC) oder Phycoerythrin (PE) gekoppelt wurde. Die auf diese Weise fluoreszenzmarkierten Zellen können dann mit bekannten Sortiertechniken nach dem Grad der Fluoreszenzstrahlung sortiert werden.

Das erfindungsgemäße Isolierverfahren kann insbesondere bei Knochenmarktransplantationen eingesetzt werden, um gesunde hämatopoetische Stammzellen von gegebenenfalls vorhandenen Tumorzellen abzutrennen und auf diese Weise das zu transplantierende Knochenmark zu reinigen. Vor allem bei Eigentransplantationen (autologen Transplantationen) im Anschluß an eine Chemo- oder Strahlentherapie wird damit die Gefahr einer Reinfektion des Patienten deutlich vermindert.

Um das Nachweis- bzw. Isolierverfahren schnell und ohne umständliche Vorbereitungen durchführen zu können, sieht die Erfindung einen entsprechenden Kit vor, der den erfindungsgemäßen Antikörper A3C6E2 enthält.

Die mit dem erfindungsgemäßen Isolierverfahren gewonnenen und dadurch gleichzeitig gereinigten Zellen können weiter subfraktioniert werden, um noch homogenere Zellpopulationen zu gewinnen. Diese Subfraktionierung kann z.B. mit Hilfe von monoklonalen Antikörpern erfolgen, die gegen ein Antigen gerichtet sind, das nur bei einer Subpopulation der den SCF-Rezeptor tragenden Zellen vorkommt. Ein solches Antigen ist z.B. das Zelloberflächenprotein CD34.

Die erfindungsgemäß isolierten, aufgereinigten hämatopoetischen Zellen eignen sich besonders für eine gentherapeutische Behandlung, die einen retroviral vermittelten Gentransfer in die hämatopoetischen Zellen umfaßt.

Die Erfindung betrifft auch die In vitro-Verwendung eines monoklonalen Antikörpers, der spezifisch an einen humanen Stammzellfaktor bindet, zur Hemmung der Hämatopoese, wobei der Antikörper von Hybridomzellen produziert und freigesetzt wird, die unter der Nummer DSM ACC 2247 bei der Deutschen Sammlung von Mikroorganismen und Zellkulturen GmbH, DSM, gemäß dem Budapester Vertrag hinterlegt sind und die Bezeichnung A3C6E2 tragen. Dies kann sowohl im Versuchslabor an Zellkulturen als auch in der ärztlichen Praxis erfolgen. Für eine Anwendung am Patienten sollte der erfindungsgemäße Antikörper mit Hilfssubstanzen vermischt sein, die seine Applikation erleichtern. Die Erfindung betrifft deshalb auch ein pharmazeutisches Mittel zur Hemmung der Hämatopoese, das den Antikörper in einer Menge enthält, die den SCF hemmt.

Es ist bekannt, daß sich viele neoplastische Zellen von normalen, d.h. gesunden Zellen unter anderem auch dadurch unterscheiden, daß sie keine SCF-Rezeptormoleküle auf der Zelloberfläche exprimieren.

Die Erfindung betrifft deshalb auch ein Verfahren zur Abtrennung normaler Zellen von neoplastischen Leukämiezellen, mit den grundsätzlichen Schritten:
1) Inkubation einer Mischung von normalen Zellen und neoplastischen Leukämiezellen mit einem Antikörper, und
2) Abtrennung der hämatopoetische Zellen von den neoplastischen Leukämiezellen anhand der unterschiedlichen Anzahl von SCF-Rezeptoren in der Plasmamembran der hämatopoetischen Zellen einerseits und der neoplastischen Leukämiezellen andererseits.

Das erfindungsgemäße Verfahren zeichnet sich dadurch aus, daß der Antikörper der monoklonale Antikörper A3C6E2 ist.

Die Abtrennung kann beispielsweise dadurch erfolgen, daß die Zellen in der Mischung zunächst mit dem erfindungsgemäßen Antikörper markiert werden, dann mit einem gegen den spenderorganismus des Antikörpers A3C6E2 gerichten, biotinylierten Antikörper gekoppelt werden und schließlich durch eine Avidinsäule laufen gelassen werden. Zellen mit einer hohen Anzahl von SCF-Rezeptormolekülen werden in der Säule zurückgehalten, während Zellen ohne SCF-Rezeptoren durch die Säule hindurchlaufen. Alternative Abtrennverfahren sind die direkte Immunadhärenz, die fluoreszenzaktivierte Zellsortierung (FACS) und die magnetisch aktivierte Zellsortierung (MACS).

Die vorliegende Erfindung betrifft auch ein Verfahren zum Nachweis von SCF-Rezeptoren in einer Zellprobe. Dieses Verfahren umfaßt die grundsätzlichen Schritte:
1) Inkubation einer Zellprobe mit einem Antikörper, und
2) Erfassung des an den SCF-Rezeptor gebundenen Antikörpers,
und zeichnet sich dadurch aus, daß der Antikörper der erfindungsgemäße monoklonale Antikörper A3C6E2 ist.

Die Zellprobe kann aus normalen Zellen und/oder Leukämiezellen und/oder Zellen von Lymphomen bestehen. Die Erfassung des gebundenen Antikörpers erfolgt vorzugsweise vermittels eines an den Antikörper A3C6E2 gekoppelten Markers.

Mit Hilfe dieses erfindungsgemäßen Verfahrens ist es möglich, beispielsweise eine Blutprobe oder Gewebeprobe von einem Patienten mit Verdacht auf Tumorerkrankung auf das Vorhandensein von neoplastischen oder Lymphomzellen zu testen.

Der erfindungsgemäße Antikörper A3C6E2, wie er von den unter der Nummer DSM ACC 2247 bei der Deutschen Sammlung von Mikroorganismen und Zellkulturen GmbH, DSM hinterlegten Hybridomzellen produziert und freigesetzt wird, kann ebenfalls zur Modifizierung der Sensitivität von Patienten für zell-zyklusspezifische chemotherapeutische Mittel verwendet werden.

Durch Verabreichung des erfindungsgemäßen Antikörpers in einer Menge, die die Bindung von SCF an seinen Rezeptor beschränkt oder vollständig verhindert, wird das Wachstum und die Entwicklung der den SCF-Rezeptor tragenden Zellen gehemmt.

Um die Verabreichung des erfindungsgemäßen Antikörpers zu erleichtern, sollte der Antikörper mit entsprechend geeigneten Hilfssubstanzen in einer pharmazeutischen Zubereitung vermischt sein. Die Erfindung betrifft deshalb auch ein pharmazeutisches Mittel zur Modifizierung der Sensitivität von Patienten für zellzyklus-spezifische chemotherapeutische Behandlungen, das den Antikörper A3C6E2 enthält, wie er von den unter der Nummer DSM ACC 2247 bei der Deutschen Sammlung von Mikroorganismen und Zellkulturen GmbH, DSM hinterlegten Hybridomzellen produziert und freigesetzt wird.

Das betreffende pharmazeutische Mittel sollte den Antikörper in einer Menge enthalten, die die Bindung und/oder die Produktion von SCF hemmt.

Weitere Vorteile ergeben sich aus der nachstehenden Beschreibung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird im folgenden anhand von Anwendungs- und Ausführungsbeispielen unter Heranziehung der Figuren näher erläutert.

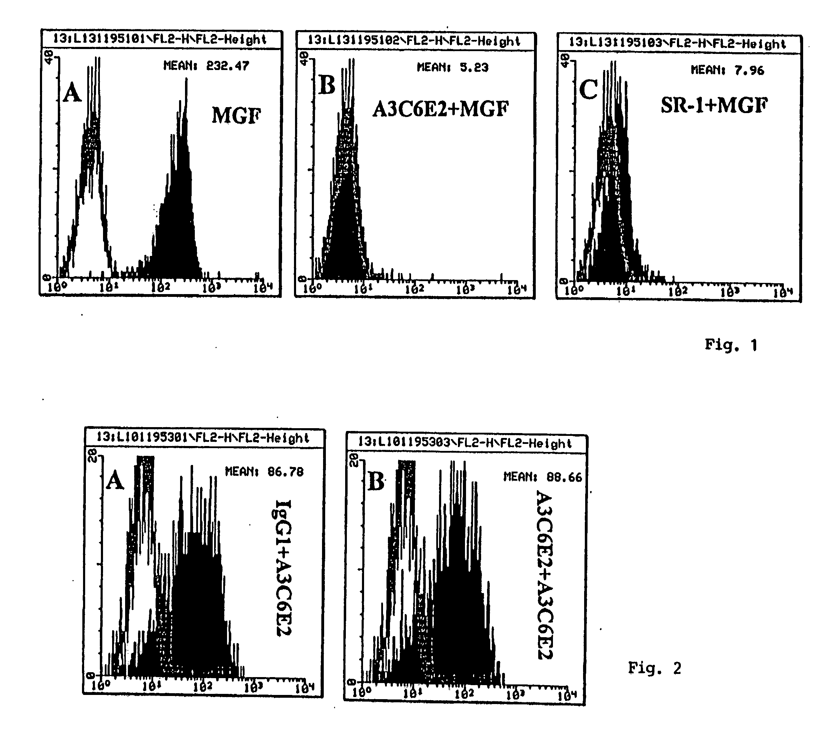

- Fig. 1: umfaßt drei Histogramme von Durchflußzytometermessungen (Untersuchung der Blockierung von biotinyliertem Liganden) an M07e-Zellen, die bei 1A nur mit Ligand (biotinylierter SCF + SA-PE) inkubiert, bei 1B mit dem Antikörper A3C6E2 vor- und mit dem Ligand nachinkubiert und bei 1C mit dem Vergleichsantikörper SR-1 vor- und mit dem Ligand nachinkubiert wurden;
- Fig. 2: umfaßt zwei Histogramme von Durchflußzytometermessungen (Untersuchung der Downregulierung des Rezeptors) an M07e-Zellen, die bei 2A mit einem IgG1-Kontrollantikörper vorinkubiert und dann mit dem Antikörper A3C6E2 und einem anti-IgG1-PE-Antiserum nachinkubiert wurden, und die bei 2B mit dem Antikörper A3C6E2 vorinkubiert und anschließend mit demselben Antikörper und einem anti-IgG1-PE-Antiserum nachinkubiert wurden; und

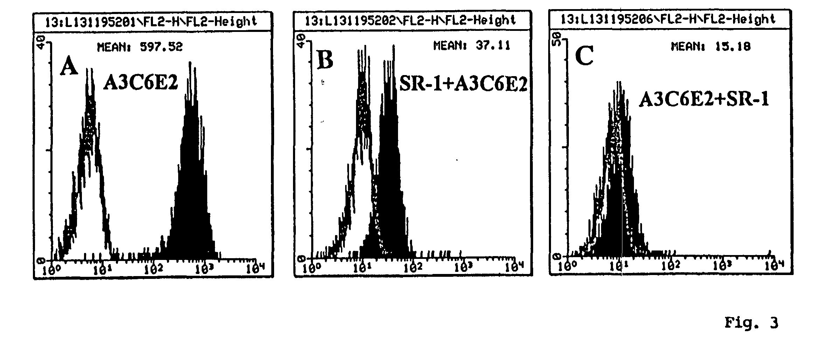

- Fig. 3: umfaßt drei Histogramme von Durchflußzytometermessungen (Epitopanalyse) an M07e-Zellen, die bei 3A mit dem Antikörper A3C6E2 inkubiert und mit einem anti-IgG1-PE-Antiserum markiert wurden, bei 3B mit dem Vergleichsantikörper SR-1 vor- und dann wie bei 3A weiterinkubiert und markiert wurden, und die bei 3C zuerst mit dem Antikörper A3C6E2 und dann mit dem Vergleichsantikörper SR-1 nachinkubiert und einem anti-IgG2A-PE-Antiserum markiert wurden.

### Beispiel 1: Herstellung und Charakterisierung von monoklonalen Antikörpern gegen das SCF - Rezeptorprotein CD117

Als Antigen werden Zellen der undifferenzierten, megakaryoblastären Zellinie MOLM-1 verwendet (Matsuo Y, Adachi T, Tsubota T, Imanishi J, Minowada J. Establishment and characterization of a novel megakaryoblastoid cell line, MOLM-1, from a patient with chronic myelogenous leukemia. Human Cell 1991; 4: 261-264).

Acht Wochen alte Balb/c-Mäuse werden zweimal in Intervallen von 10 Tagen intraperitoneal mit 10⁷ Zellen der Zellinie MOLM-1 immunisiert. Vier Tage vor der Fusion werden 5 x 10⁵ Zellen direkt in die Milz appliziert, um die Immunantwort zu verstärken.

Die Antikörper-Bildung im Mausorganismus wird dadurch überprüft, daß das Blutserum des betreffenden Tieres in dem dem Fachmann geläufigen ELISA-Test auf Bindungseigenschaften mit dem Antigen untersucht wird.

Nach ca. 3 Wochen werden die Lymphozyten des erfolgreich immunisierten Tieres gewonnen, indem die Milz herausoperiert und zu einer Zellsuspension zerkleinert wird.

Die suspendierten Milzzellen werden in Anwesenheit von Polyethylenglykol mit Myelomzellen des bekannten Stammes SP2/0 fusioniert. Die Fusionskultur wird in Hypoxanthin-, Aminopterinund Thymidin-(HAT-)haltigem Medium, hier in HAT-RPMI-1640, kultiviert, in dem sich nur Hybridzellen vermehren können, da diese sowohl die Eigenschaft der Myelomzellen zur unbegrenzten Teilungsfähigkeit als auch die Eigenschaft der antikörperproduzierenden Lymphozyten zum Wachstum in HAT-haltigem Medium haben.

Nach der Fusion werden die Zellen in Napfplatten ausplattiert und bei 37 °C und 5 % CO₂ inkubiert.

Die Kulturüberstände werden nach 10-14 Tagen auf der MOLM-1 Zellinie im Durchflußzytometer gescreent. In einem zweiten Schritt werden die Überstände auf Reaktivität mit peripheren Blutzellen getestet, da diese keine selektiven Stammzellantigene exprimieren. Überstände, die eine negative oder schwache Reaktion mit peripheren Blutzellen zeigen, werden anschließend auf Reaktivität mit Knochenmarkzellen getestet. Hybridome, die Antikörper mit Spezifität für Knochenmarkzellen produzieren, werden ausgewählt und nach dem bekannten Grenzverdünnungsverfahren vereinzelt und kultiviert, d.h. kloniert.

Bei dieser Screening-Strategie wird Nutzen aus der Tatsache gezogen, daß im Knochenmark verstärkt undifferenzierte Zellen einschließlich von hämatopoetischen Stammzellen vorkommen.

Positiv reagierende Hybridomzellkulturen werden weiter kultiviert, die Antikörper angereichert, gereinigt und charakterisiert.

Nach der obigen Screening-Strategie wurde der monoklonale Antikörper A3C6E2 erhalten. Die Isotype wurde über PE-konjugierte anti-Isotyp-spezifische Antiseren durch direkte Immunfluoreszenz zu IgG1 bestimmt.

Die Produktion, Reinigung und Charakterisierung der Antikörper erfolgte mit den in Fachkeisen allgemein bekannten Methoden.

Der Antikörper A3C6E2, der von den unter der Nummer DSM ACC 2247 bei der Deutschen Sammlung von Mikroorganismen und Zellkulturen GmbH, DSM, hinterlegten Hybridomzellen erzeugt wird, weist die folgenden charakteristischen Merkmale auf:

| | |
|---|---|
| Immunglobulinklasse | IgG1 |
| spezifische Bindungsaffinität an | CD117 |

### Beispiel 2: Identifizierung des von dem monoklonalen Antikörper A3C6E2 erkannten Antigens.

Die Identifizierung des Antigens erfolgte durch Bindungsversuche an Maus-Zellen mit und ohne humane SCF-Rezeptoren (Transfektanten).

Eine Probe (A) mit Mausfibroblasten der Zellinie NIH-3T3 / hckit, die mit dem humanen c-kit-Gen transfiziert sind (Yarden et al., EMBO J. 6: 3341, 1987), wurde mit dem erfindungsgemäßen Antikörper A3C6E2 inkubiert.

Zur Kontrolle wurde eine Probe (B) mit Mausfibroblasten der nicht transfizierten Zellinie NIH-3T3 ebenfalls mit dem Antikörper A3C6E2 in derselben Konzentration wie Probe (A) inkubiert.

Beide Proben wurden mit einem anti-IgG1-PE-Antiserum markiert und anschließend im Durchflußcytometer analysiert.

Eine Bindung des Antikörpers A3C6E2 konnte ausschließlich bei Probe (A) nachgewiesen werden. Das heißt, daß der erfindungsgemäße Antikörper A3C6E2 spezifisch an diejenigen Zellen gebunden hat, die das c-kit-Gen aufweisen und infolgedessen den SCF-Rezeptor exprimieren.

### Beispiel 3: Identifizierung des monoklonalen Antikörpers A3C6E2 als antagonistisch (den Liganden blockierend) aber nicht agonistisch (den Liganden simulierend) wirkender Antikörper

Zur Blockierung der Ligandenbindung wurde eine Probe (1A) M07e-Zellen mit dem biotinylierten Liganden MGF-Biotin (MGF-b = kit Ligand) in der Konzentration 200ng/ml inkubiert und dann mit SA-PE markiert (Positivkontrolle).

Eine zweite Probe (1B) M07e-Zellen wurde zunächst 30 Minuten mit dem Antikörper A3C6E2 in der Konzentration 7µg/ml vorinkubiert und anschließend wie Probe (1A) behandelt.

Beide Proben wurden im Durchflußcytometer analysiert. Die Ergebnisse sind in Fig 1A und Fig. 1B dargestellt.

Als Vergleichsversuch wurde eine dritte Probe (1C) M07e-Zellen wie Probe (A) behandelt mit dem Unterschied, daß anstelle des erfindungsgemäßen Antikörpers A3C6E2 der bekannte Antikörper SR-1 in einer Konzentration von 10µg/ml eingesetzt wurde.

Wie aus den Histogrammen in Fig.1 hervorgeht, blockiert der erfindungsgemäße Antikörper A3C6E2 die Bindung des Liganden zu 99, 99% und der Vergleichsantikörper SR-1 blockiert zu 98,7%.

Die durch die Bindung des Liganden initiierte Aktivierung des Rezeptors führt bei Tyrosin-Kinase-Rezeptoren wie dem SCF-Rezeptor zunächst zur Bildung von Rezeptordimerkomplexen und deren Internalisierung in die Zelle. Manche Antikörper können den Liganden simulieren, d.h. sie wirken agonistisch, und führen deshalb ebenfalls zu einer solchen Rezeptordimer-Bildung und - Internalisierung (Bühring et al., Cancer Res. 53: 4424, 1993) .

Eine Probe (2A) MOLM-1-Zellen wurde 2 Std. bei 37°C mit einem Kontroll-IgG1-Antikörper vorinkubiert, dann mit dem erfindungsgemäßen Antikörper A3C6E2 inkubiert und schließlich mit anti-IgG1-PE-Antiserum nachinkubiert.

Eine zweite Probe (2B) wurde zunächst mit dem Antikörper A3C6E2 inkubiert und anschließend wie Probe (2A) behandelt.

Beide Proben wurden im Durchflußcytometer analysiert. Die dabei erhaltenen Histogramme sind in Fig. 2 dargestellt. Beide Histogramme zeigen gleichstarke Signale. Das bedeutet, daß keine Internalisierung des Rezeptors stattgefunden hat, und daß der erfindungsgemäße Antikörper A3C6E2 nicht agonistisch wirkt, d.h. den Liganden nicht simuliert, aber stark antagonistisch (blockierend) wirkt.

### Beispiel 4: Verwendung des monoklonalen Antikörpers A3C6E2 zur Hemmung der Bindung von SR-1

Eine Probe (3A) M07e-Zellen wurde zunächst mit dem Antikörper A3C6E2 inkubiert und der gebundene Antikörper dann mit einem anti-IgG1-PE-Antiserum markiert.

Eine zweite Probe (3B) M07e-Zellen wurde zuerst mit dem bekannten Antikörper SR-1 in der Konzentration 10µg/ml 30 Minuten vorinkubiert und anschließend wie Probe (3A) mit dem Antikörper A3C6E2 und dem anti-IgG1-PE-Antiserum behandelt.

Eine dritte Probe (3C) wurde zuerst 30 Minuten mit dem erfindungsgemäßen Antikörper A3C6E2 vorinkubiert, dann mit dem bekannten Antikörper SR-1 nachinkubiert und der gebundene SR-1 Antikörper schließlich mit einem anti-IgG2A-PE-Antiserum markiert.

Alle drei Zellproben wurden im Durchflußcytometer analysiert. Die dabei gewonnenen Histogramme sind in den Figuren 3A, 3B und 3C dargestellt.

Wie aus diesen Histogrammen hervorgeht, wurde in Probe (3B) die Bindung des Antikörpers A3C6E3 zu 94% durch den Antikörper SR-1 gehemmt und in Probe (3C) die Bindung von SR-1 zu 98,7% durch A3C6E2 inhibiert.

Diese Ergebnisse zeigen, daß beide Antikörper das gleiche oder zumindest ein sehr ähnliches Epitop auf dem SCF-Rezeptorprotein CD117 erkennen.

## Patentansprüche

1. Monoklonaler Antikörper, der spezifisch an einen humanen Stammzellfaktor (SCF) - Rezeptor bindet, **dadurch gekennzeichnet, daß** er von Hybridomzellen produziert und freigesetzt wird, die unter der Nummer DSM ACC 2247 bei der Deutschen Sammlung von Mikroorganismen und Zellkulturen GmbH, DSM, gemäß dem Budapester Vertrag hinterlegt sind und die Bezeichnung A3C6E2 tragen.

2. Hybridomzellen, **dadurch gekennzeichnet, daß** sie unter der Nummer DSM ACC 2247 bei der Deutschen Sammlung von Mikroorganismen und Zellkulturen GmbH, DSM, gemäß dem Budapester Vertrag hinterlegt sind und die Bezeichnung A3C6E2 tragen.

3. *In vitro*-Verwendung des Antikörpers nach Anspruch 1 in einem Verfahren zur Diagnose von Tumoren, insbesondere von Leukämie und von Lymphomen.

4. Pharmazeutisches Mittel zur diagnostischen Behandlung von Tumoren, insbesondere von Leukämie und von Lymphomen, **dadurch gekennzeichnet, daß** es den Antikörper gemäß Anspruch 1 enthält.

5. Pharmazeutisches Mittel nach Anspruch 4, **dadurch gekennzeichnet, daß** der Antikörper an ein Diagnostikum gekoppelt ist.

6. Pharmazeutisches Mittel zur therapeutischen Behandlung von Tumoren, insbesondere von Leukämie und Lymphomen, **dadurch gekennzeichnet, daß** es den Antikörper nach Anspruch 1 enthält.

7. Pharmazeutisches Mittel nach Anspruch 6, **dadurch gekennzeichnet, daß** der Antikörper mit einer wirksamen Menge eines Therapeutikums gekoppelt ist, das gegen die Leukämiezellen und/oder die Lymphomzellen gerichtet ist.

8. In vitro-Verwendung des Antikörpers nach Anspruch 1 zum Nachweis und/oder zur Isolierung von hämatopoetischen Zellen.

9. Verfahren zum Nachweis und/oder zur Isolierung von hämatopoetischen Zellen mit den Schritten:
(a) Inkubation einer Zellsuspension, die hämatopoetische Zellen enthält, mit einem Antikörper, der an hämatopoetische Zellen bindet, und
(b) Abtrennung der den Antikörper bindenden Zellen von den übrigen Zellen,
**dadurch gekennzeichnet, daß** der Antikörper der monoklonale Antikörper gemäß Anspruch 1 ist.

10. Verfahren zum Nachweis und/oder zur Isolierung von hämatopoetischen Zellen nach Anspruch 9, **dadurch gekennzeichnet, daß** die Abtrennung mittels Säulenchromatographie erfolgt.

11. Verfahren zum Nachweis und/oder zur Isolierung von hämatopoetischen Zellen nach Anspruch 9, **dadurch gekennzeichnet, daß** die Abtrennung mittels fluoreszenzaktivierter Zellsortierung (FACS) erfolgt.

12. Verfahren zum Nachweis und/oder zur Isolierung von hämatopoetischen Zellen nach Anspruch 9, **dadurch gekennzeichnet, daß** die Abtrennung durch direkte Immunadhärens erfolgt.

13. Kit zum Nachweis von hämatopoetischen Zellen, **dadurch gekennzeichnet, daß** er den Antikörper nach Anspruch 1 enthält.

14. In vitro-Verwendung des Antikörpers nach Anspruch 1 zur Isolierung und Aufreinigung von hämatopoetischen Zellen für eine gentherapeutische Behandlung, die einen retroviral vermittelten Gentransfer in die hämatopoetischen Zellen umfaßt.

15. *In vitro*-Verwendung eines monoklonalen Antikörpers, der spezifisch an einen humanen Stammzellfaktor (SCF)-Rezeptor bindet, zur Hemmung der Hämatopoese, **dadurch gekennzeichnet, daß** der Antikörper von Hybridomzellen produziert und freigesetzt wird, die unter der Nummer DSM ACC 2247 bei der Deutschen Sammlung von Mikroorganismen und Zellkulturen GmbH, DSM, gemäß dem Budapester Vertrag hinterlegt sind und die Bezeichnung A3C6E2 tragen.

16. Pharmazeutisches Mittel zur Hemmung der Hämatopoese, **dadurch gekennzeichnet, daß** das Mittel den monoklonalen Antikörper, der von Hybridomzellen produziert und freigesetzt wird, die unter der Nummer DSM ACC 2247 bei der Deutschen Sammlung von Mikroorganismen und Zellkulturen GmbH, DSM, gemäß dem Budapester Vertrag hinterlegt sind und die Bezeichnung A3C6E2 tragen, in einer Menge enthält, die die Bindung des Stammzellfaktors (SCF) hemmt.

17. Verfahren zur Abtrennung normaler Zellen von neoplastischen Leukämiezellen mit den Schritten:
(a) Inkubation einer Mischung von normalen Zellen und neoplastischen Leukämiezellen mit einem Antikörper, und
(b) Abtrennung der hämatopoetische Zellen von den neo-plastischen Leukämiezellen anhand der unterschiedlichen Anzahl von Stammzellfaktor (SCF)-Rezeptoren in der Plasmamembran der hämatopoetischen Zellen einerseits und der neoplastischen Leukämiezellen andererseits,
**dadurch gekennzeichnet, daß** der Antikörper der monoklonale Antikörper gemäß Anspruch 1 ist.

18. Verfahren zum Nachweis von Stammzellfaktor (SCF)-Rezeptoren in einer Zellprobe mit den Schritten:
(a) Inkubation einer Zellprobe mit einem Antikörper, und
(b) Erfassung des an den Stammzellfaktor (SCF) - Rezeptor gebundenen Antikörpers,
**dadurch gekennzeichnet, daß** der Antikörper der monoklonale Antikörper nach Anspruch 1 ist.

19. Verfahren nach Anspruch 18, **dadurch gekennzeichnet, daß** die Erfassung des gebundenen Antikörpers vermittels eines an den Antikörper gekoppelten Markers erfolgt.

20. zu Verfahren nach einem der Ansprüche 18 oder 19, **dadurch gekennzeichnet, daß** die Zellprobe aus normalen Zellen und/oder Leukämiezellen und/oder Zellen von Lymphomen besteht.

21. Pharmazeutisches Mittel zur Modifizierung der Sensitivität von Patienten für zellzyklus-spezifische chemotherapeutische Behandlungen, **dadurch gekennzeichnet, daß** es den Antikörper nach Anspruch 1 enthält.

22. Pharmazeutisches Mittel nach Anspruch 21, **dadurch gekennzeichnet, daß** das Mittel den Antikörper in einer Menge enthält, die die Bindung des Stammzellfaktors (SCF) hemmt.

## Claims

1. A monoclonal antibody that binds specifically to a human stem cell factor (SCF) receptor, **characterized in that** the antibody is produced and released by hybridoma cells that were deposited, under No. DSM ACC 2247, at the Deutsche Sammlung von Mikroorganismen und Zellkulturen GmbH, DSM, in accordance with the Budapest Treaty, and which are designated A3C6E2.

2. Hybridoma cells, **characterized in that** they are deposited under No. DSM ACC 2247, at the Deutsche Sammlung von Mikroorganismen und Zellkulturen GmbH, DSM, in accordance with the Budapest Treaty, and which are designated A3C6E2.

3. Use of the antibody according to claim 1 *in vitro* in a method for the diagnosis of tumors, especially of leukemia and lymphoma.

4. A pharmaceutical means for diagnostic treatment of tumors, especially of leukemia and lymphoma, **characterized in that** it contains an antibody according to claim 1.

5. The pharmaceutical means according to claim 4, **characterized in that** the antibody is coupled to a diagnostic agent.

6. A pharmaceutical means for therapeutical treatment of tumors, especially of leukemia and lymphoma, **characterized in that** contains the antibody according to claim 1.

7. The pharmaceutical means according to claim 6, **characterized in that** the antibody is coupled to an effective quantity of a therapeutic agent directed against the leukemia cells and/or the lymphoma cells.

8. Use of the antibody according to claim 1 *in vitr*o for detecting and/or isolating hematopoietic cells.

9. A method for detecting and/or isolating hematopoietic cells, comprising the steps of:
(a) Incubating a cell suspension containing hematopoietic cells with a monoclonal antibody that binds to hematopoietic cells, and
(b) separating the cells to which the antibody has bound, from the remaining cells,
**characterized in that** the antibody is the monoclonal antibody according to claim 1.

10. The method for detecting and/or isolating hematopoietic cells according to claim 9, **characterized in that** the separation is carried out by means of column chromatography.

11. The method for detecting and/or isolating hematopoietic cells according to claim 9, **characterized in that** the separation is carried out by means of fluorescence-activated cell sorting (FACS).

12. The method for detecting and/or isolating hematopoietic cells according to claim 9, **characterized in that** the separation is carried out by direct immune adherence.

13. A kit for detecting hematopoietic cells, **characterized in that** it contains an antibody according to claim 1.

14. Use of the antibody according to claim 1 *in vitro* for isolating and purifying hematopoietic cells for gene therapeutic treatment comprising a retrovirally mediated gene transfer into the hematopoietic cells.

15. Use of an antibody in vitro that binds specifically to human stem cell factor (SCF) receptor for inhibition of hematopoiesis, **characterized in that** the antibody is produced and released by hybridoma cells that were deposited, under No. DSM ACC 2247, at the Deutsche Sammlung von Mikroorganismen und Zellkulturen GmbH, DSM, in accordance with the Budapest Treaty, and which are designated A3C6E2.

16. A pharmaceutical means for inhibition of hematopoiesis, **characterized in that** the agent contains the antibody that is produced and released by hybridoma cells that were deposited under No. DSM ACC 2247, at the Deutsche Sammlung von Mikroorganismen und Zellkulturen GmbH, DSM, in accordance with the Budapest Treaty, and which are designated A3C6E2, in a quantity that inhibits binding of the stem cell factor (SCF).

17. A method for separating normal cells from neoplastic leukemia cells comprising the steps of:
(a) Incubating a mixture of normal cells and neoplastic leukemia cells with an antibody, and
(b) separating the hematopoietic cells from the neoplastic leukemia cells, according to the different amounts of stem cell factor (SCF) receptors in the plasma membrane of the hemapoietic cells on the one hand and the neoplastic leukemia cells on the other hand,
**characterized in that** the antibody is the monoclonal antibody according to claim 1.

18. A method for detecting stem cell factor (SCF) receptors in a cell sample comprising the steps of:
(a) Incubating a cell sample with an antibody, and
(b) ascertaining the antibody bound to the stem cell factor (SCF) receptor,
**characterized in that** the antibody is the antibody according to claim 1.

19. The method according to claim 18, **characterized in that** ascertainment of the bound antibody is carried out by means of a marker coupled to the antibody.

20. The method according to claim 18 or 19, **characterized in that** the cell sample consists of normal cells and/or leukemia cells and/or lymphoma cells.

21. A pharmaceutical means for modification of the sensitivity of patients to cell cycle-specific chemotherapeutic treatment **characterized in that** it contains the antibody according to claim 1.

22. The pharmaceutical means according to claim 21, **characterized in that** it contains the antibody in a quantity which inhibits the binding of the stem cell factor (SCF)

## Revendications

1. Anticorps monoclonal, qui se lie spécifiquement sur un récepteur humain de facteur de stimulation de cellules souches (SCF), **caractérisé en ce qu'**il est produit et libéré par des cellules d'hybridome, qui ont été déposées sous le numéro DSM ACC 2247 auprès de la Deutsche Sammlung von Mikroorganismen und Zellkulturen GmbH, conformément au traité de Budapest et qui portent la dénomination A3C6E2.

2. Cellules d'hybridome, **caractérisées en ce qu'**elles ont été déposées sous le numéro DSM ACC 2247 auprès de la Deutsche Sammlung von Mikroorganismen und Zellkulturen GmbH, conformément au traité de Budapest et **en ce qu'**elles portent la dénomination A3C6E2.

3. Utilisation *in vitro* de l'anticorps selon la revendication 1 dans un procédé pour le diagnostic de tumeurs, en particulier de leucémies et de lymphomes.

4. Agent pharmaceutique pour le traitement diagnostique de tumeurs, en particulier de leucémies et de lymphomes, **caractérisé en ce qu'**il contient l'anticorps selon la revendication 1.

5. Agent pharmaceutique selon la revendication 4, **caractérisé en ce que** l'anticorps est couplé à un diagnostic.

6. Agent pharmaceutique pour le traitement thérapeutique de tumeurs, en particulier de leucémies et de lymphomes, **caractérisé en ce qu'**il contient l'anticorps selon la revendication 1.

7. Agent pharmaceutique selon la revendication 6, **caractérisé en ce que** l'anticorps est couplé à une quantité active d'un agent thérapeutique, qui est dirigé contre les cellules de leucémie et/ou les cellules de lymphome.

8. Utilisation *in vitro* de l'anticorps selon la revendication 1, pour la détection et/ou pour l'isolement de cellules hématopoïétiques.

9. Procédé pour la détection et/ou pour l'isolement de cellules hématopoïétiques, comprenant les étapes :
(a) d'incubation d'une suspension cellulaire, qui contient des cellules hématopoïétiques, avec un anticorps, qui se lie aux cellules hématopoïétiques, et
(b) de séparation des cellules se liant à l'anticorps des autres cellules,
**caractérisé en ce que** l'anticorps est l'anticorps monoclonal selon la revendication 1.

10. Procédé pour la détection et/ou pour l'isolement de cellules hématopoïétiques selon la revendication 9, **caractérisé en ce que** la séparation est réalisée par chromatographie sur colonne.

11. Procédé pour la détection et/ou pour l'isolement de cellules hématopoïétiques selon la revendication 9, **caractérisé en ce que** la séparation est réalisée par triage des cellules activées par fluorescence (FACS).

12. Procédé pour la détection et/ou pour l'isolement de cellules hématopoïétiques selon la revendication 9, **caractérisé en ce que** la séparation est réalisée par adhésion immune directe.

13. Kit de détection de cellules hématopoïétiques, **caractérisé en ce qu'**il contient l'anticorps selon la revendication 1.

14. Utilisation *in vitro* de l'anticorps selon la revendication 1, pour l'isolement et la purification de cellules hématopoïétiques pour un traitement par thérapie génique, qui comprend un transfert de gène dans les cellules hématopoïétiques par un moyen rétroviral.

15. Utilisation *in vitro* d'un anticorps monoclonal, qui se lie spécifiquement sur un récepteur humain de facteur de stimulation de cellules souches (SCF), pour l'inhibition de l'hématopoïèse, **caractérisée en ce que** l'anticorps est produit et libéré par des cellules d'hybridome, qui ont été déposées sous le numéro DSM ACC 2247 auprès de la Deutsche Sammlung von Mikroorganismen und Zellkulturen GmbH, conformément au traité de Budapest et qui portent la dénomination A3C6E2.

16. Agent pharmaceutique pour l'inhibition de l'hématopoïèse, **caractérisé en ce que** l'agent contient l'anticorps monoclonal, qui est produit et libéré par des cellules d'hybridomes, qui ont été déposées sous le numéro DSM ACC 2247 auprès de la Deutsche Sammlung von Mikroorganismen und Zellkulturen GmbH, conformément au traité de Budapest et qui portent la dénomination A3C6E2, en une quantité qui inhibe la liaison du facteur de stimulation des cellules souches (SCF).

17. Procédé de séparation de cellules normales et de cellules leucémiques néoplasiques, comprenant les étapes :
(a) d'incubation d'un mélange de cellules normales et de cellules leucémiques néoplasiques avec un anticorps, et
(b) de séparation des cellules hématopoïétiques et des cellules leucémiques néoplasiques sur la base du nombre différent de récepteurs du facteur de stimulation des cellules souches (SCF) dans la membrane plasmique des cellules hématopoïétiques d'une part, et des cellules leucémiques néoplasiques d'autre part,
**caractérisé en ce que** l'anticorps est l'anticorps monoclonal selon la revendication 1.

18. Procédé de détection de récepteurs du facteur de stimulation de cellules souches (SCF) dans un échantillon cellulaire, comprenant les étapes :
(a) d'incubation d'un échantillon cellulaire avec un anticorps, et
(b) de détermination de l'anticorps lié au récepteur du facteur de stimulation de cellules souches (SCF),
**caractérisé en ce que** l'anticorps est l'anticorps monoclonal selon la revendication 1.

19. Procédé selon la revendication 18, **caractérisé en ce que** la détermination de l'anticorps lié est réalisée à l'aide d'un marqueur conjugué à l'anticorps.

20. Procédé selon la revendication 18 ou 19, **caractérisé en ce que** l'échantillon cellulaire consiste en des cellules normales et/ou des cellules leucémiques et/ou des cellules de lymphome.

21. Agent pharmaceutique pour modifier la sensibilité de patients à un traitement chimiothérapeutique spécifique du cycle cellulaire, **caractérisé en ce qu'**il contient l'anticorps selon la revendication 1.

22. Agent pharmaceutique selon la revendication 21, **caractérisé en ce que** l'agent contient l'anticorps en une quantité qui inhibe la liaison du facteur de stimulation des cellules souches (SCF).
